# EUROPEAN PATENT SPECIFICATION

(11) **EP 2 462 941 B1**
(45) Date of publication and mention of the grant of the patent: **23.12.2015**
(21) Application number: 10472002.4
(22) Date of filing: 04.10.2010
(51) Int. Cl.: A61K 36/88

(54) **Method and installation for extraction of plant raw material**
Verfahren und Installation zur Extraktion von Rohmaterial von Pflanzen
Procédé et installation pour l'extraction de matériaux bruts de plantes

(30) Priority: 20.09.2010 BG 11075410
(43) Date of publication of application: 13.06.2012
(73) Proprietor: R&D Ltd., Sofia 1000 (BG)
(72) Inventor: Lubenova, Shopova Daniela, Sofia (BG)

(56) References cited:
- WO-A1-2010/000070
- SELLES M ET AL: "Quantitative evaluation of galanthamine and related alkaloids in wild plants and tissue cultures of Narcissus confusus by high performance liquid chromatography", ANALUSIS, SOCIETE DE PRODUCTIONS DOCUMETAIRES, RUEIL-MALMAISON, FR, vol. 25, no. 5, 1 January 1997 (1997-01-01), pages 156-158, XP009158962, ISSN: 0365-4877
- B&#XFFFD;ATRICE KAUFMANN ET AL: "Recent extraction techniques for natural products: microwave-assisted extraction and pressurised solvent extraction", PHYTOCHEMICAL ANALYSIS, vol. 13, no. 2, 1 January 2002 (2002-01-01), pages 105-113, XP55014298, ISSN: 0958-0344, DOI: 10.1002/pca.631
- TOMASZ ET AL.: "Pressurized liquid extraction and anticholinesterase activity-based thin-layer chromatography with bioautography of Amaryllidaceae alkaloids", ANALYTICA CHIMICA ACTA, vol. 633, no. 2, February 2009 (2009-02), pages 188-196, XP025880103, ISSN: 0003-2670, DOI: DOI:10.1016/J.ACA.2008.11.053
- FULZELE D P ET AL: "Comparison of techniques for the extraction of the anti-cancer drug camptothecin from Nothapodytes foetida", JOURNAL OF CHROMATOGRAPHY, ELSEVIER SCIENCE PUBLISHERS B.V, NL, vol. 1063, no. 1-2, 21 January 2005 (2005-01-21), pages 9-13, XP004702297, ISSN: 0021-9673, DOI: 10.1016/J.CHROMA.2004.11.020
- MA W ET AL: "Application of ionic liquids based microwave-assisted extraction of three alkaloids N-nornuciferine, O-nornuciferine, and nuciferine from lotus leaf", TALANTA, ELSEVIER, AMSTERDAM, NL, vol. 80, no. 3, 1 January 2010 (2010-01-01), pages 1292-1297, XP026801331, ISSN: 0039-9140 [retrieved on 2009-09-19]
- ISTATKOVA RALITSA ET AL: "ULTRASOUND- AND MICROWAVE-ASSISTED EXTRACTIONS OF ALKALOIDS FROM MEDICINAL PLANTS", DOKLADI NA BOLGARSKATA AKADEMIYA NA NAUKITE, vol. 62, no. 8, 2009, pages 935-940, XP009158947, ISSN: 1310-1331

## Description

### TECHNICAL FIELD

This invention relates to a method for extraction of plant raw material, from plant species of the Amaryllidaceae, for obtaining alkaloids that find application as medicines, cosmetics and food supplements, and it also relates to an installation for extraction of plant raw material.

### BACKGROUND ART

Alkaloids are known to be widely used separately in pure form and as a total extract, which justifies the development of numerous methods for their isolation from a number of natural sources, in particular from plant raw material.

The various plant species contain different quantity of varied alkaloids, which requires a specific approach and development of an appropriate method for isolation.

Literature sources reveal different technologies based on the principle of solid-liquid extraction of alkaloids. Thus, for example, a method for isolation of alkaloids from the Amaryllidaceae is described in BG 25140, according to which the milled drug is alkalized and subjected to extraction with water at 30-40°C and pH 9-11. The total alkaloid is subjected to treatment for separation of individual alkaloids. The extraction is multi-step and the extract obtained has low alkaloid content.

A method for extraction of alkaloids for preparation of galanthamine from the same plant source is described in GB 938767, where the plant raw material, after alkalization with ammonia, is subjected to extraction with a water non-miscible organic solvent. This extraction is also a multi-step one and the extract obtained has low alkaloid content. The operation involves work with highly toxic solvents.

Obtaining alkaloids by extraction with water, low alcohols or their aqueous mixtures is described in GB 942200. The extraction is incomplete and results in losses of valuable raw material.

Obtaining of alkaloids from galantamine-containing plants by means of alkaline solutions of inorganic bases or water miscible organic solvents, as well as water non-miscible solvents, is described in international application WO 2006/063666. The extraction is performed at a temperature ranging from 20°C to the boiling temperature of the solvent, followed by separation of the complex mixture of alkaloids and the relevant extractant.

The known methods of solid-liquid extraction have long duration of the technological process, use large quantities of raw material and solvents, pollute to a great rate the environment in their industrial application and the extract obtained is with a high rate of impurities.

Furthermore, it is known that microwave radiation at a certain step of the extraction process is used in a number of cases of extraction of biological substances from plant raw material. The analysis of the patent situation in the world shows that various methods with or without solvents have been developed and specific microwave radiation regimen and extraction conditions have been established for the different plant sources and obtained products.

In patent EP 0 485 668 is described a method of microwave extraction of plant raw material, using non-polar extractant penetrable or partially penetrable to microwave radiation, The extractant together with the raw material is fed in the extractor, and is subjected to microwave radiation after which the processed material is separated and the extract obtained is subjected to evaporation and condensation,. An installation including devices for feeding the non-polar extractant and the raw material, a mixer connected to them to which an extractor, a separator, an evaporator and a condenser are connected in a sequence, a microwave source being connected to the extractor, is also described. The described method and installation are intended for recovery of essential oils and terpenes, in which organic solvents and other toxic reagents are used.

From CN 1239504 (C) is known a method for extraction of alkaloids from plant species of the Amaryllidaceae, in particular from tubers of the said plant species, in which the plant material dried to a definite degree is placed in a microwave oven and is subjected to microwave radiation. After that, the radiated powdered raw material is taken out from the microwave oven and is subjected to extraction with a solvent selected from water, acid solution or an organic solvent. The extraction is performed upon heating on a reflux or stirring at room temperature, after which filtration of the extract is performed. The microwave radiation frequency is over 300 MHz, the power is 50-50000 W, and the time for microwave radiation is from 0,5 to 120 min.

This method requires separate equipment - for microwave radiation and for accomplishment of the extraction.

It is also stated in patent CN 1239504 that there are reports of alkaloid isolation applying microwave radiation to the plant raw material. However, further on in the text there is a description of a method for biochemical product obtaining in general. This method includes powdering of the plant raw material, mixing with water or an organic solvent, subjection of the mixture to microwave radiation. Then, separation of the solid material from the liquid, removal of the solvent and isolation of the active ingredients follows. It is noted that a disadvantage of this method is that the solvent is placed in the microwave oven together with the raw material and the organic solvent is subjected to the microwave radiation, which results in consumption of particularly large quantities of the solvent, but also this method causes serious pollution of the environment, and besides a serious fire hazard exists. It is also pointed out that there are no reports in patent and non-patent literature of the application of this method for alkaloid obtaining from plants of the Amaryllidaceae.

An extractor for isolation of valuable components from plant and animal raw materials is known from patent RU 2 283 161 (C2). The extractor has a body, an open-top lidded cylinder positioned coaxially to it, a pressure generator with pressure ranging from heightened to lowered pressure, and the relevant pipelines, extract collector, low and high pressure receivers, controllable valves, an extractant tank, a piston pump for extractant injection, nozzle for the extractant, microwave radiation sources, a super high frequency oscillator, a power unit and a control unit.

The said extractor is complicated for execution, maintenance and control. Besides, it is not designated for obtaining specifically of alkaloids from plant raw material.

Considering the known methods for alkaloid obtaining, and in particular from the Amaryllidaceae, using microwave radiation, the problems that should be solved are to achieve a quantitative obtaining alkaloids from plant raw material, in particular from plant species of the Amaryllidaceae, which is energetically economic, and yet no harmful solvents to be used, to achieve a maximum degree of isolation, to reduce the process duration, to ensure ecological cleanliness of the entire manufacture and microbial purity of the extract. It is necessary to create an installation for accomplishment of extraction of plant raw material, including an extractor and a microwave source, which is compact, with minimum number of equipment and which allows intensive mass exchange process and automatic process control.

### TECHNICAL DESCRIPTION OF THE INVENTION

According to the invention, a method has been created for extraction of plant raw material for obtaining alkaloids, including at least one extraction with extractant of alkaline aqueous solution and application of microwave radiation of the plant material during extraction and subsequent separation of the plant material and extract that in a given case is subjected to concentration, where the microwave radiation parameters being as follows: power from 0,850 to 2,55 kW, frequency - 2450 MHz, duration up to 120 min and temperature from 30 to 35°C. The extraction is conducted in a regimen of periodic forced circulation of the liquid, and the extract is filtered, enters the circulation contour and returns to the extractor. Preferably, the plant raw material is the aerial part of Amaryllidaceae plants, in particular of Leucojum aesivum, Galantus nivalis, Galantus elewesii, and Narcissus species. The solvent is selected from aqueous solution of calcium carbonate, ammonia, sodium hydroxide, the ratio of plant raw material: extractant being from 1:4 to 1:20. After completion of the extraction, the exhausted plant raw material is taken out of the extractor and the extract obtained is taken out necessary to create an installation for accomplishment of extraction of plant raw material, including an extractor and a microwave source, which is compact, with minimum number of equipment and which allows intensive mass exchange process and automatic process control.

### TECHNICAL DESCRIPTION OF THE INVENTION

According to the invention, a method has been created for extraction of plant raw material for obtaining alkaloids, including at least one extraction with alkaline aqueous extractant and application of microwave radiation of the plant material during extraction and subsequent separation of the plant material and extract that in a given case is subjected to concentration, where the microwave radiation parameters being as follows: power from 0,850 to 2,55 kW, frequency - 2450 MHz, duration up to 120 min and temperature from 30 to 35°C. The extraction is conducted in a regimen of periodic forced circulation of the liquid, and the extract is filtered, enters the circulation contour and returns to the extractor. Preferably, the plant raw material is the aerial part of Amaryllidaceae plants, in particular of Leucojum aesivum, Galantus nivalis, Galantus elewesii, and Narcissus species. The alkaline aqueous extractant is selected from aqueous solution of calcium carbonate, ammonia, sodium hydroxide, the ratio of plant raw material: extractant being from 1:4 to 1:20. After completion of the extraction, the exhausted plant raw material is taken out of the extractor and the extract obtained is taken out of the circulation contour, thus avoiding the necessity of additional separation and inclusion of one more device for the purpose. The temperature, extraction time, heating and recirculation time are automatically controlled.

The installation for accomplishment of the method according to the invention comprises: a tank for a solvent, connected to an extractor placed in a chamber, a microwave source-magnetron is assembled to the chamber wall. The bottom part of the extractor ends in an outlet for the extract to the circulation contour, which is connected to the top part of the extractor. A filter, a temperature sensor, a pump, a drain valve, a circulation valve and a pH meter are fitted in the circulation contour. An inner perforated-wall container, connected to a device for carrying in and taking out of the extractor, is positioned in the extractor. The temperature sensor, pump, drain valve and circulation valve are connected to a control unit.

### Description of the enclosed figure:

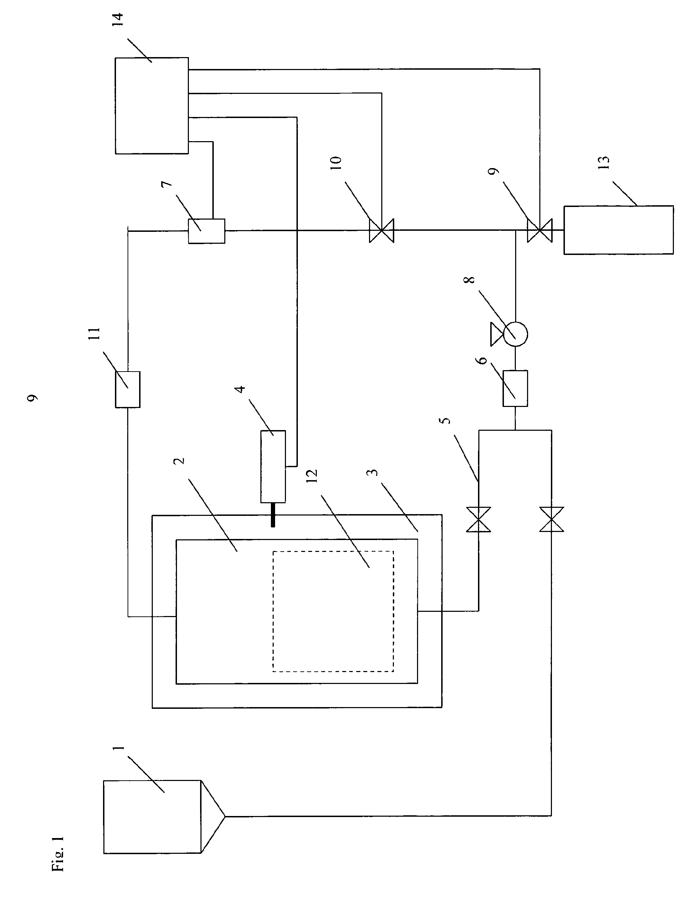

Fig. 1 presents schematically the installation according to the invention.

### Detailed description of embodiment of the installation for extraction of plant raw material:

Fig.1 illustrates schematically the installation. It comprises: a tank for the solvent (1), connected to an extractor (2) that is placed in a chamber (3), a microwave source- magnetron (4) is assembled to the wall of chamber (3). Circulation contour (5) is connected to the outlet in the bottom part of extractor (2). The circulation contour (5) also is connected to the top part of the extractor (2). A filter (6), a temperature sensor (7), a pump (8), a drain valve (9), a circulation valve (10) and a pH meter (11) are fitted in the circulation contour (5). Inner perforated-wall container (12), connected to a device for carrying in and taking out of the extractor (not shown on the figure), is positioned in the extractor. The circulation contour is connected through a drain valve (9) to an extract collector (13). The temperature sensor (7), pump (8), drain valve (9) and circulation valve (10) are connected to control unit (14).

The installation works as follows: The previously prepared dried plant material is placed in the perforated-wall container (12) outside the extractor (2), the container (12) is lowered in the extractor (2), the solvent is fed, the microwave source is turned on. While the extraction is running, the liquid is led out of the extractor at specified intervals of time through a filter (6) and is fed into the circulation contour (5). Circulation is regulated by a valve (10). After completion of extraction, the microwave source is turned off, the extract is drained through a filter (6) and a valve (9) into a collector (13) and the container (12) is taken out of the extractor.

The installation works as follows: The previously prepared dried plant material is placed in the perforated-wall container (12) outside the extractor (2), the container (12) is lowered in the extractor (2), the solvent is fed, the microwave source is turned on. While the extraction is running, the liquid is led out of the extractor at specified intervals of time through a filter (6) and is fed into the circulation contour (5). Circulation is regulated by a valve (10). After completion of extraction, the microwave source is turned off, the extract is drained through a filter (6) and a valve (9) into a collector (13) and the container (12) is taken out of the extractor.

### Examples of embodiment of the method for extraction of plant raw material:

### Example 1

3 kg of dried and previously milled plant raw material from aerial parts of summer snowflake Leocojum aestivum L. of the Amaryllidaceae, with high content of the alkaloid galantamine, are fed in the extractor (2) by means of the perforated-wall container (12). 25 litres of alkaline aqueous extractant of calcium carbonate are added in the extractor (2). The microwave radiation source (4) is turned on, by which radiation with frequency of 2450 MHz, power 2,55 kW is performed for 30 min. The solution recirculates for 3 min every 10 min in the circulation contour (5). Temperature is maintained at 32°C. After completion of extraction, the extract obtained, through the filter (6) by means of the pump (8) and through the drain valve (12), enters the collector (13). The plant raw material is again poured with alkaline solution and a second extraction is performed under the same conditions. Both extracts are united. The alkaloid content is more than 0,05 %, and the galantamine content is more than 80 %. Yield of alkaloids - 99 %.

### Example 2

3 kg of dried and previously milled plant raw material from aerial parts of summer snowflake Leocojum aestivum L. of the Amaryllidaceae, with high content of alkaloids, mainly of the alkaloid N-demethylgalantamine, are fed in the extractor (2) by means of the perforated-wall container (12). Alkaline aqueous extractant of ammonia is added in the extractor (2). The microwave radiation source (4) is turned on, by which radiation with frequency of 2450 MHz, power 1,7 kW is performed for 30 min. The solution recirculates for 3 min every 10 min in the circulation contour (5). Temperature is maintained at 30°C. After completion of extraction, the extract obtained, through the filter (6) by means of the pump (8) and through the drain valve (12), enters the collector (13). The plant raw material is again poured with alkaline solution of ammonia and a second extraction is performed under the same conditions. Both extracts are united. The alkaloid content is more than 0,05 %, and the galantamine content is below 10%. And N-demethylgalantamine - more than 80 %. Yield of alkaloids - 99%.

### Example 3

3 kg of dried and previously milled tubers from Narcissus pseudonarcissus of the Amaryllidaceae are fed in the extractor (2) by means of the perforated-wall container (12). Alkaline aqueous extractant of calcium carbonate is added in the extractor (2). The microwave radiation source (4) is turned on by means of which radiation with frequency of 2450 MHz, power 2,55 kW is performed for 50 min. The solution recirculates for 3 min every 10 min in the circulation contour (5). Temperature is maintained at 32°C. After completion of extraction, the extract obtained, through the filter (6) by means of the pump (8) and through the drain valve (12), enters the collector (13). The plant raw material is again poured with alkaline solution and a second extraction is performed under the same conditions. Both extracts are united. The alkaloid content is more than 0,05 %, and the galantamine content is more than 80 %. Yield of alkaloids - 99%.

## Claims

1. Method for extraction of plant raw material from a plant species of the family *Amaryllidaceae,* for alkaloid obtaining, by means of solid-liquid extraction using microwave radiation, following by separation, **characterised in that** it includes performance of at least one extraction with alkaline aqueous extractant, selected from aqueous calcium carbonate, ammonia and sodium hydroxide with the ratio of plant raw material **from a species of *Amaryllidaceae*** : extractant being from 1:4 to 1: 20 and application of microwave radiation of the plant material and extractant during extraction at frequency 2450 MHz, power 0,850 - 2,55 kW, duration up to 120 min and temperature from 30 to 35°C, in a regimen of periodic forced circulation, where after completion of extraction, separation of the plant material and the extract is accomplished by leading the extract out of the extractor and taking the exhausted plant material out of the extractor.

2. Method for extraction of plant raw material according to claim 1, **characterised in that** the plant species of the family of the *Amaryllidaceae* is *Leucojum aestivum, Galanthus* nivalis *Galanthus* elwesii or *Narcissus.*

3. Method for extraction of plant raw material according to one of the claims from 1 to 2, **characterised in that** the aerial part of the plants is used as a plant material.

4. Method for extraction of plant raw material according to one of the claims from 1 to 2, **characterised in that** plant tubers are used as plant material.

5. Installation for accomplishment of the method for extraction of plant raw material, according to claims 1- 4, comprising an extractor in which coaxially positioned an inner perforated-wall container is connected to a device for carrying in and taking out of the extractor, and at least one microwave source, **characterised in that** it contains a tank for solvent (1), connected to an extractor (2) that is placed in a chamber (3), the microwave source-magnetron (4) is assembled to the chamber wall (3), to the outlet in the bottom part of the extractor (2) the circulation contour (5) is connected, which is also connected with the top part of the extractor (2), and in the circulation contour (5) a filter (6), a temperature sensor (7), a pump (8), a drain valve (9), a circulation valve (10) and a pH meter (11) are fitted, at which in the extractor (2) an inner perforated-wall container (12) is placed, and the circulation contour (5) is connected through a drain valve (9) to the collector for the extract obtained (13), and the temperature sensor (7), pump (8), drain valve (9) and circulation valve (10) are connected to the control unit (14).

## Patentansprüche

1. Verfahren für die Extraktion von Pflanzenrohstoff aus der Pflanzenart der Familie Amaryllidaceae zur Gewinnung von Alkaloiden durch eine Fest-flüssig-Extraktion mittels Mikrowellenradiation und anschließender Separation. Das Verfahren charakterisiert sich dadurch, dass es die Durchführung wenigstens einer Extraktion mit einem alkalischwässrigem Extragent einschließt. Beim Auswählen des Extragents werden folgende Stoffe berücksichtigt: Calciumhyrogencarbonat, Ammoniak und Natriumhydroxid bei einem Verhältnis des Pflanzenstoffs der Familie Amaryllidaceae wie folgt : Extragent von 1:4 bis 1:20 und Anwendung einer Mikrowellenradiation des Pflanzenmaterials und des Extragents während der Extraktion bei einer Frequenz von 2450 MHz, Kapazität 0,850 - 2,55 kW, Zeitdauer bis 120 Min. und einer Temperatur von 30 bis 35° C unter einer periodischen Zwangszirkulation, wobei nach Beendigung der Extraktion die Separation des Pflanzenmaterials und des Extrakts durch Ausführung des Extrakts aus dem Extraktor und Entzug des abfallenden Pflanzmaterials dem Extraktor.

2. Verfahren für die Extraktion von Pflanzenrohstoff gemäß dem Anspruch 1., das sich dadurch charakterisiert, dass die Pflanzenart der Familie Amaryllidaceae Leucojum aestivus, Galanthus niuvals, Galantus elwesii oder Narcissus darstellt.

3. Verfahren für die Extraktion von Pflanzenrohstoff gemäß einem der Ansprüche 1. bis 2., das sich dadurch charakterisiert, dass als Pflanzenmaterial der über der Erde gelegene Teil der Pflanzen benutzt wird.

4. Verfahren für die Extraktion von Pflanzenrohstoff, das sich dadurch charakterisiert, dass als Pflanzenmaterial die Pflanzenknollen benutzt werden.

5. Eine Anlage zur Realisierung des Verfahrens für die Extraktion von Pflanzenrohstoff gemäß den Ansprüchen 1. bis 4., welche folgende Geräte einschließt: Extraktor, worin ein koaxial positionierter Innenbehälter, dessen Wände perforiert sind, an eine Einrichtung zum Ein- und Ausführen des Pflanzenmaterials in und aus dem Extraktor angeschlossen ist, sowie wenigstens eine Mikrowellenquelle. Die Anlage charakterisiert sich dadurch, dass sie aus folgenden Bestandteilen besteht: einem in einer Schachtel (3) gelegenen und zum Extraktor (2) angeschlossenen Behälter (1) für das Lösungsmittel; einer Mikrowellenquelle, (Magnetron) (4), die an der Wand der Schachtel (3) eingebaut ist; einem Zirkulationsgerät (Kontur) (5), das an den Bodenausgang des Extraktors (2) angeschlossen ist. Das Zirkulationsgerät ist auch an den obersten Teil des Extraktors (2) angeschlossen. Im Zirkulationsgerät (5) sind folgende Bestandteile eingebaut; der Filter (6), der Temperaturfühler (7), die Pumpe (8), die Ablassungsklappe (9), die Zirkulationsklappe (10) und das pH-Messgerät (11). Dabei ist im Extraktor (2) ein Innenbehälter mit perforierten Wänden (12) gelegt, und das Zirkulationsgeät (5) ist über eine Ablassungsklappe an den Kollektor für das erhaltene Extrakt (13) angeschlossen. Der Temperaturfühler (7), die Pumpe (8), die Ablassungsklappe (9) und die Zuirkulationsklappe (10) sind an den Kontrollblock (14) angeschlossen.

## Revendications

1. Méthode pour l'extraction de matière première végétale de plante de la famille des Amaryllidaceae, pour obtenir des alcaloïdes, par extraction solide-liquide à l'aide de micro-ondes, suivie de la séparation, **caractérisée par le fait qu'**elle inclut au moins une extraction avec un agent d'extraction aqueux-alcalin, sélectionné parmi le carbonate de calcium, l'ammoniac et l'hydroxyde de sodium avec un rapport de matière première végétale à partir **d'une espèce de Amaryllidaceae:** agent d'extraction de 1: 4 à 1: 20 et l'application de irradiation micro-ondes de la matière végétale et de l'agent d'extraction pendant l'extraction à une fréquence de 2450 MHz, puissance 0,850 - 2,55 kW, d'une durée de jusqu'à 120 min et une température de 30 à 35 °C, dans un régime de circulation forcé périodique, dans lequel après achèvement de l'extraction, la séparation de la matière végétale et l'extrait est obtenue par l'élimination de l'extrait de l'extracteur et l'élimination du matériel végétal traite de l'extracteur.

2. Méthode pour l'extraction de matière première végétale selon la demande 1, **caractérisée par le fait que** l'espèce de plante de la famille des Amaryllidaceae est le Leucojum aestivum, le niuvalis Galanthus, le Galanthus elwesii ou le Narcisse

3. Procédé d'extraction de matière première végétale selon l'une demandes 1 à 2, **caractérisée par le fait que** la partie aérienne des plantes est utilisée comme matériel végétal.

4. Procédé d'extraction de matière première végétale selon l'une demandes 1 à 2, ***caractérisée par le fait que** tubercules de plantes sont utilisées comme matériel végétal.*

5. Installation pour la réalisation du procédé d'extraction de matière première végétale, conforme les demandes 1- 4, comprenant un extracteur qui est positionné de façon coaxiale dans un récipient interne à parois perforés, relié à un dispositif permettant la introduction dans et l'extraction hors de l'extracteur et au moins d'une source micro-onde, **caractérisée par le fait que** 'il contient un réservoir de solvant (1), relié à un extracteur (2) qui est placé dans une boite (3), une source de microondes - magnétron (4) est assemblé à la paroi de la boite (3), à l'orifice dans la partie inférieure de l'extracteur (2) le contour de circulation (5) est connecté, qui est relié à la partie supérieure de l'extracteur (2), et dans le contour de circulation (5) sont fixes un filtre (6), un capteur de température (7), une pompe (8), une soupape de vidange (9), une soupape de circulation (10) et un pH-mètre (11) dans lequel cas dans l'extracteur (2) est placé un conteneur récipient interne à parois perforés (12) et le contour de circulation (5) est relié à une soupape de vidange (9) au collecteur pour l'extrait obtenu (13), et le capteur de température (7), la pompe (8), la vanne de vidange (9) et la soupape de circulation (10) sont reliés à l'unité de commande (14).
